# EUROPÄISCHE PATENTANMELDUNG

(11) **EP 1 536 230 A1**
(43) Veröffentlichungstag der Anmeldung: **01.06.2005**
(21) Anmeldenummer: 03090412.2
(22) Anmeldetag: 28.11.2003
(51) Int. Cl.: G01N 33/543, G01N 33/68

(54) **Durchflusszytometrischer Partikel-basierter Bindungsassay**

(71) Anmelder: Deutsches Rheuma-Forschungszentrum Berlin, 10117 Berlin (DE)
(72) Erfinder: Baumgrass, Ria, Dr., 14558 Nuthetal (DE); Blex, Christian, 12587 Berlin (DE)
(74) Vertreter: Rasch, Dorit

(57) **Zusammenfassung**

Verfahren zur Detektion einer transienten Protein-Protein-Wechselwirkung in einer Probe dadurch gekennzeichnet, dass ein erstes Protein markiert und ein zweites Protein mit einem Tag kombiniert wird, die Proteine mit einem anti-Tag-beschichteten Partikel in Kontakt gebracht, inkubiert und durchflusszytometrisch analysiert werden und die Detektion der Markierung auf den Partikeln ein Hinweis auf die Protein-Protein-Wechselwirkung ist.

## Beschreibung

Die Erfindung betrifft ein Verfahren zur Detektion von Protein-Protein-Wechselwirkungen, insbesondere einer Calcineurin-NFAT-Wechselwirkung, sowie einen Kit und dessen Verwendung zur Detektion von Protein-Protein-Wechselwirkungen.

Wechselwirkungen von biologischen Molekülen sind als permanente oder transiente Wechselwirkungen möglich. Es sind zahlreiche permanente Wechselwirkungen von biologischen Strukturen bekannt, wie z.B. Antikörper-Antigen-Wechselwirkungen oder Rezeptor-Ligand-Wechselwirkungen, wobei die Strukturen - Antikörper und Antigen oder Rezeptor und Ligand - eine hohe Affinität zueinander aufweisen und stabil sowie stark wechselwirken. Die hohe Affinität ist für Folgeprozesse wie z.B. die Internalisierung - von Rezeptor und Ligand - oder andere Folgereaktionen erforderlich. Sofern die wechselwirkenden Strukturen keine hohe Affinität zueinander aufweisen, liegt eine transiente Wechselwirkung vor, die von den genannten Wechselwirkungen, wie beispielsweise der Rezeptor-Ligand-Wechselwirkung, völlig verschieden ist. Eine Internalisierung der interagierenden Partner erfolgt bei der transienten Wechselwirkung von z.B. Proteinen nicht. Die einzelnen Wechselwirkungen (permanent und transient) können mittels einem K_{d}-Wert voneinander abgrenzt werden. Durch den K_{d}-Wert wird die Protein-Protein-Interaktion bzw. die Assoziierung zwischen den Proteinen über das Massenwirkungsgesetz bestimmt. Der K_{d}-Wert ist eine Equilibriumsdissoziationskonstante, die diejenige Konzentration angibt, bei der bei der Hälfte der Proteine eine Wechselwirkung und Bindung erfolgt ist. Der K_{d}-Wert ist reziprok zur Affinität, die die Stärke der Bindung zwischen den Proteinen kennzeichnet. Transiente Protein-Protein-Wechselwirkungen weisen eine Dissoziationskonstante von K_{d} ≥ 10⁻⁸ auf, die ein flexibles Wechselspiel der Proteine - wie z.B. binden und wiederholtes lösen - erlaubt. Bei der starken oder stabilen, permanenten Wechselwirkung liegen die Werte im Bereich von K_{d} ≤ 10⁻⁹. Nach einer erfolgten Bindung trennen sich die Partner hierbei nicht so, dass sie in ihrer weitestgehend ursprünglichen Form wieder vorliegen.

Transiente Protein-Protein-Wechselwirkungen sind Schlüsselphänomene in der Bildung funktioneller Proteinkomplexe und ein wichtiger Bestandteil der dynamischen Signalübertragung in der Zelle. Die Protein-Protein-Wechselwirkung erfolgt in der Regel so, dass kleine Peptidmotive des einen Proteins an einer Docking-Domäne eines anderen Proteins binden.

Neben Docking-Domänen, die in mehreren Proteinen vorkommen, z. B. SH3 oder WW Domänen, gibt es auch spezifische Docking-Sites für spezielle Proteininteraktionen.

Die Funktionen der transienten Protein-Protein-Wechselwirkungen können sehr unterschiedlich sein. So kann z. B. die Bindung eines Proteins an sogenannte Ankerproteine (AKAPs) eine subzelluläre Lokalisation bestimmen. Des Weiteren ist für viele Enzyme die Bindung ihrer Proteinsubstrate außerhalb des aktiven Zentrums eine Voraussetzung für ihre enzymatische Umsetzung. Auch bei der Signalübertragung in der Zelle kann die Interaktion von Signalkettengliedern die Signalweiterleitung fördern oder unterbrechen. Auf Grund der herausgestellten Bedeutung von transienten Protein-Protein-Interaktionen für die Regulation verschiedenster Prozesse sind Untersuchungen zum Mechanismus und zur Modellierung solcher Interaktionen von außerordentlichem Interesse für die Forschung, Klinik und Wissenschaft, insbesondere für die pharmakologische Forschung beispielsweise bei der Drug-Entwicklung.

Aus diesem Grund sind im Stand der Technik mehrere Methoden zur Untersuchung von Protein-Protein-Wechselwirkungen offenbart worden. Die bekannten Methoden lassen sich unterteilen in
(i) molekularbiologische Methoden,
(ii) genetische Methoden und
(iii) biochemisch-physikalische Methoden.

Bei der molekularbiologischen Methode werden DNA-Bibliotheken nach Genen oder Genfragmenten durchsucht, deren Produkte mit einem Zielprotein interagieren können. Beispiele hierfür sind Yeast Two-Hybrid-Systeme oder der Phage Display.

Bei der genetischen Methode werden synthetische Mutanten mit definierten Eigenschaften gesucht. Biochemischphysikalische Methoden umfassen beispielsweise die Protein-Affinitätschromatographie, die Co- Immunopräzipitation (pull-down-assay), die Methode des in vivo und in vitro crosslinking von Bindungspartnern sowie die Chip-Technologie, z. B. Peptid Arrays oder Biacore-Surface Plasmon Resonanz.

Mit Erfolg werden bisher insbesondere molekularbiologische und genetische Methoden zum Screening von Bindungspartnern eingesetzt. Für Screening-Untersuchungen von Proteinbindungsstellen sind hingegen die biochemisch-physikalischen Methoden besser geeignet als die molekularbiologischen oder genetischen, da mit ihnen ein höherer Probendurchsatz (z. B. beim high-through-put-screening) analysiert werden kann.

Die klassischen Methoden wie beispielsweise die Protein-Affinitätschromatographie, die pull-down-assays und das crosslinking sind auf Grund ihrer Aufwändigkeit und die Vielzahl der einzelnen Verfahrensschritte den Chip-Technologien unterlegen. Allerdings sind für ein high-through-put-screening (HTS) nach einer niedermolekularen Verbindung - d. h. einem potentiellen Drug -, die die Protein-Protein-Wechselwirkung aufhebt, die Chipsysteme sehr material- und zeitintensiv und meistens nicht computergestützt durchführbar.

Aufgabe der Erfindung war es daher, ein Verfahren zur Detektion von transienten Protein-Protein-Wechselwirkungen bereitzustellen, welches insbesondere als high-through-put-screening eingesetzt werden kann. Das heißt unter anderem, dass es automatisierbar ist, dass es im Mikrotiterplattenformat und als homogener Assay durchgeführt werden kann. Weiterhin war es Aufgabe der Erfindung, ein Verfahren bereitzustellen, das sensitiv, spezifisch, robust, gut reproduzierbar und schnell und außerdem kostengünstig durchführbar ist sowie eine einfache Auswertung und eine breite Anwendbarkeit auf Grund flexibler Verfahrensparameter ermöglicht.

Die Erfindung löst diese Aufgabe durch Bereitstellung eines Verfahrens zur Detektion einer transienten Protein-Protein-Wechselwirkung (K_{d} ≥ 10⁻⁸) in einer Probe, wobei ein erstes Protein mit einer Markierung und ein zweites Protein mit einem Tag kombiniert - z. B. biotinyliert - werden, die Proteine mit anti-Tag-beschichteten - z.B. Streptavidin-beschichteten - Partikel in Kontakt gebracht, inkubiert und durchflusszytometrisch analysiert werden und die Detektion der Markierung auf den Partikeln ein Hinweis auf die Protein-Protein-Wechselwirkung ist, wobei die Protein-Protein-Wechselwirkung von einer permanenten Wechselwirkung insbesondere einer Antikörper-Antigen- oder einer Rezeptor-Ligand-Wechselwirkung als Form der stabilen, nicht-transienten Wechselwirkung verschieden ist.

Es war überraschend, dass es möglich ist, die transiente Protein-Protein-Wechselwirkung durchflusszytometrisch zu bestimmen. Die nicht-stabile, transiente Wechselwirkung (K_{d} ≥ 10⁻⁸) zwischen zwei Proteinen galt als nicht so stark, als dass diese, im Gegensatz zur permanenten, starken oder stabilen Wechselwirkung (K_{d} ≤ 10⁻⁹), durchflusszytometrisch und partikelassoziiert bestimmt werden könnte. Die Entwicklung der Technik der Detektion der transienten Wechselwirkung verlief in eine andere Richtung, nämlich in die Richtung der Yeast Two-Hybrid-Systeme, des Phage Display und pull-down-assay sowie weiterer molekularbiologischer und genetischer Methoden. Durch die anmeldungsgemässe entwicklungsraffende Leistung wird eine Fehlvorstellung revidiert und das seit langem ungelöste Bedürfnis einer einfachen, sicheren und effizienten Bestimmung der transienten Proteinwechselwirkung befriedigt. Der anmeldungsgemässe Lösungsvorschlag erscheint aus rückblickender Betrachtungsweise einfach. Die einfache Lösung ist jedoch nicht gleichbedeutend mit auf der Hand liegend, da es nicht einleuchtend ist, dass gerade eine so einfache, ein lange bestehendes Bedürfnis befriedigende und erfolgreiche Maßnahme zur Bestimmung der transienten Protein-Protein-Wechselwirkung nicht schon längst verwirklicht wurde.

Protein-Protein-Wechselwirkungen oder Interaktionen sind spezifische Wechselwirkungen zwischen zwei oder mehreren Proteinen, die hinsichtlich ihrer physikalischen Eigenschaften der Wechselwirkungen klassifiziert sind: stark und permanent gegenüber schwach und transient. Die Stärke der Wechselwirkungen wird bestimmt durch die Kräfte, die die Protein-Protein-Interaktion vermitteln. Es sind elektrostatische Wechselwirkungen, Wasserstoffbrückenbindungen, van der Waals Kräfte und hydrophobe Effekte. Die Stärke der Wechselwirkungen wird charakterisiert durch die Gleichgewichtskonstante der Dissoziation K_{d}, die in biologischen Prozessen extrem breit ist und einen Bereich von 10⁻⁴ bis 10⁻¹⁴ M umfasst.

Starke d.h. stabile Protein-Protein-Interaktionen haben einen K_{d} ≤ 10⁻⁹, während schwache, d.h. transiente Protein-Protein-Wechselwirkungen einen K_{d} ≥ 10⁻⁸ besitzen. In biologischen Systemen werden die Interaktionen zwischen Proteinen stark reguliert und andere Größen außer dem K_{d} - Wert sind für die Dauer der Wechselwirkungen in Zellen ebenfalls entscheidend, wie z.B. die Stabilität und die subzelluläre Lokalisation. Die *in vitro* Bestimmung der Gleichgewichtskonstante der Dissoziation ist bei K_{d} ≤ 10⁻⁹ , d.h. bei starker Interaktion der Proteine, z.B. mittels Radioisotopmarkierung oder ELISA-Techniken möglich. Ist jedoch die Gleichgewichtskonstante der Dissoziation bei K_{d} ≥ 10⁻⁸, d.h. ist die Interaktion der Proteine nur transient, so muss man Techniken verwenden, bei denen das Gleichgewicht der Bindungspartner nicht beeinflusst wird, wie z.B. die Gleichgewichtszentrifugation.

Effektive Antigen-Antikörper- und Rezeptor-Ligand-Interaktionen haben K_{d} ≤ 10⁻⁹ und sind damit permanent. Die physiologische Aufgabe beider Komplexe erfordert es, dass sie möglichst stabil sind und nicht gelöst werden. Die Bindung von Antikörpern an Antigene soll dem Körper die Entfernung der Antigene aus dem Körper erleichtern. Durch den Prozess der Rekombination während der AK-Bildung wird der Antikörper für eine permanente Bindung optimiert. Die Optimierung der Rezeptor-Ligand-Interaktionen erfolgte während der Evolution. Eine permanente Bindung von Rezeptor und Ligand ist für die Internalisierung des Komplexes und die Desensitivierung der Zellen für diesen Liganden notwendig.

Demgegenüber sind Protein-Protein-Interaktionen mit einem Kd ≥ 10⁻⁸ nur transient. Die physiologischen Aufgaben solcher Komplexe sind vielfältig. Eine wichtige Funktion ist z.B. die Gewährleistung der Signalweiterleitung in Zellen. Dafür muss die Assoziation und Dissoziation von Proteinen flexibel auf ein Signal reagieren können. Dies ist bei K_{d}-Werten im micro- und millimolarem Bereich gegeben. Das Netzwerk der Signalweiterleitung ist ein gut abgestimmtes und innerhalb von wenigen Sekunden bis Minuten reagierendes System von Prozessen, wie z.B. Protein-Protein-Wechselwirkungen, Phosphorylierungen und Dephosphorylierungen.

Eine Probe im Sinne der Erfindung ist eine Bezeichnung für ein wässriges System, dessen Beschaffenheit chemisch, biologisch, klinisch oder ähnlich geprüft werden kann.

Die beiden Proteine können im Sinne der Erfindung sämtliche Moleküle sein, die im Wesentlichen aus Aminosäuren bestehen. Das heißt, Proteine im Sinne der Erfindung sind auch Strukturen, die weniger als 100 bzw. 50 Aminosäuren umfassen und somit auch als Peptide bezeichnet werden können. Peptide und Proteine werden demgemäss im Zusammenhang mit der Erfindung synonym verwendet. Selbstverständlich können die Proteine andere Strukturen wie Lipide oder Kohlenhydrate aber auch artifizielle oder natürliche Aminosäure- oder nicht-Aminosäurebausteine umfassen.

Das erste Protein kann beispielsweise mit einem Fluoreszenzmarker wie FITC markiert werden. Das zweite Protein kann mit jedem Tag kombiniert werden, der/das eine Bindung an die Partikel erlaubt. Ein Tag im Sinne der Erfindung ist ein Protein-Anhängsel oder ein Peptid bzw. eine andere Struktur, die mit dem zu untersuchenden Protein kombiniert, beispielsweise fusioniert, wird. Das zweite Protein kann beispielsweise biotinyliert vorliegen und somit Biotin als Tag aufweisen. Dem Fachmann sind weitere Tag-Strukturen oder Tags aus Katalogen und Standardwerken der Biochemie bekannt. Dieses Tag am zweiten Protein besitzt eine hohe Affinität zu einer anti-Tag Substanz oder einem anti-Tag an oder auf den Partikeln.

Die Proteine bzw. Peptide werden vor, nach oder bei der Markierung und der Kombination mit dem Tag mit Partikeln in Kontakt gebracht, die mit anti-Tag-Strukturen so modifiziert, insbesondere beschichtet sind, dass sie mit dem jeweiligen Tag interagieren und binden können. Dem Fachmann sind derartige Tag/anti-Tag Paare bekannt, d.h. die Auswahl des Tags bestimmt die Struktur des anti-Tags, ohne dass eine erfinderische Auswahl durch einen Fachmann erforderlich ist oder das dieser bei der Ausführung der erfindungsgemässen Lehre zunächst eine technische Aufgabe lösen muss. Wenn das zweite Protein biotinyliert vorliegt und somit Biotin als Tag aufweist, ist die anti-Tag-Struktur Streptavidin, d.h. Streptavidin beschichtete Partikel. Weitere bevorzugte Tag/anti-Tag Paare sind: Protein A/ Immunglobulin, GST (Glutathion-S-Transferase)/ Glutathion, Pin-Point (in vivo Biotinylierung)/ Avidin, Thioredoxin/ Thiobond, PET (Cellulose-Binde-Domäne)/ Cellulose und/oder PMal (Maltose-Binde-Domäne)/ Amylose und andere. Bevorzugte anti-Tag-beschichtete Partikel sind ausgewählt aus der Gruppe umfassend Streptavidin-, Glutathion-, Biotin-, Nickel-NTA-, Cellulose-, Amylose-, Thiobond-, Avidin- und/oder Immunglobulin-beschichtete Partikel.

Das erste und das zweite Protein sowie die beschichteten Partikeln werden inkubiert und im Anschluss durchflusszytometrisch analysiert.

Die durchflusszytometrische Bestimmung kann mit den dem Fachmann bekannten Vorrichtungen erfolgen, wie z. B. dem Fluorescence Activated Cell Sorting (FACS). Die Durchflusszytometrie ermöglicht das Zählen und die Analyse von physikalischen und molekularen Eigenschaften der Partikel in einem Flüssigkeitsstrom. Erfindungsgemäss wird bevorzugt die Fluoreszenz der Partikel mit Hilfe der Durchflusszytometrie analysiert, z.B. Luminexgerät (BioRad oder Qiagen) oder Facs Calibur. Sofern eine Fluoreszenz detektiert werden kann, ist dies ein Hinweis darauf, dass auf den Partikeln die zweiten Proteine gebunden vorliegen, wobei diese mit den ersten Protein assoziiert sind, so dass deren Fluoreszenz als indirekte Markierung der Partikel gemessen werden kann. Die Detektion der Markierung auf dem Partikeln ist demgemäss ein Hinweis auf die Proteinwechselwirkung in Form einer Bindung oder Assoziierung zwischen dem ersten und dem zweiten Protein.

Das Zustandekommen der Bindung zwischen dem ersten und dem zweiten Protein kann so z. B. erfolgen, dass beim in-Kontakt-bringen
- des ersten mit dem zweiten Protein bzw.
- des ersten und zweiten Proteins mit den anti-Tag-beschichteten Partikeln oder
- des zweiten Proteins mit den Partikeln gefolgt von einem in-Kontakt-bringen mit dem ersten Protein
eine Interaktion zwischen dem ersten und dem zweiten Protein, also zwischen den für die Wechselwirkung verantwortlichen Domänen der Proteine, stattfindet.

In der Probe können demgemäss Komplexe vorliegen, die das erste und das zweite Protein bzw. das erste und das zweite Peptid umfassen. Bei diesen Komplexen handelt es sich um das Ergebnis einer ggf. gegenseitigen Erkennung mindestens zweier Proteine und der anschließenden Bindung zwischen diesen beiden. Diese Komplexe, Assoziate bzw. Protein-Protein-Konstrukte können sich in der Probe über das zweite Protein an die Partikel anlagern, so dass mittels einer partikelassoziierten Erfassung der Fluoreszenzmarkierung die Protein-Protein-Wechselwirkung und -Bindung bestimmt und analysiert werden kann.

Es ist möglich, in den Proben einzelne Parameter wie den pH-Wert, die Protein- oder Ionen-Konzentration und ähnliches zu modifizierten, um deren Einfluss auf die Protein-Protein-Wechselwirkung zu bestimmen. Weiterhin können Moleküle wie z. B. Bindungs-Antagonisten zugesetzt werden, um zu analysieren, inwieweit diese die Protein-Protein-Wechselwirkung modifizieren, beispielsweise inhibieren.

Vorteilhafterweise kann das erfindungsgemässe Verfahren für ein high-throgh-put-screening von Substanzen verwendet werden, wie beispielsweise niedermolekulare Substanzen, um deren Fähigkeit zu testen, die Bindung zwischen den Proteinen bzw. Peptiden zu blockieren bzw. zu verstärken.

Das erste und/oder zweite Protein kann modifiziert werden, um zu bestimmen, ob die Modifikation einen Einfluss auf die Protein-Protein-Wechselwirkung hat. Die Modifikation der Proteine oder Peptide kann beispielsweise beinhalten, dass insbesondere die Bindungs-Domänen beispielsweise durch Mutationen in ihrer Struktur geändert werden.

Ein weiterer Vorteil des Verfahrens ist es, dass es in einem Mikrotiterplattenformat und/oder bevorzugt als homogener Assay durchgeführt werden kann. Die Vorteile des homogenen Assays liegen beispielsweise darin, dass keine weiteren Separationsschritte wie Zentrifugieren oder Waschen zum Detektieren der Protein-Protein-Wechselwirkung erforderlich sind. Die durchflusszytometrische Detektion erfolgt vorteilhafterweise so, dass ein gutes Signal zu Background-Verhältnis erzielt werden kann und das nicht relevante Proteine nicht detektiert werden, da nur Partikel-assoziierte Fluoreszenz erfasst oder nachgewiesen wird. Auf Grund des insgesamt robusten Aufbaus der durchflusszytometrischen Bestimmung können multiple Fehlerquellen ausgeschlossen werden. Die mit dem erfindungsgemässen Verfahren vorgenommenen Detektionen sind daher gut reproduzierbar. Bei Parallelproben wird eine Standardabweichung von max. ca. 8 % und bei Parallelansätzen von ca. max. 15 % erreicht. Auf Grund des geringen Probenvolumens wird mit Vorteil nur wenig Testsubstanz verbraucht. Da für die durchflusszytometrischen Analysen keine teuren Komponenten oder Chips benötigt werden, ist das Verfahren kostengünstig durchzuführen. Durchflusszytometrische Bestimmungen erfolgen sehr schnell; im Regelfall wird eine Versuchsdauer von einer Stunde nicht überschritten. Das flexible Format der Durchflusszytometer gestattet eine freie Veränderung des Mess-Systems. Außerdem sind die generierten Daten leicht auszuwerten, da eine Verschiebung der mittleren Fluoreszenzintensität im Vergleich als Kontrolle verwendet werden kann.

Es ist durch die genannten Vorteile möglich, Protein-Protein-Wechselwirkungen mit hoher Zuverlässigkeit und einer Ersparnis an Zeit, Material, Arbeitsstufen und Kosten insbesondere als high-trough-put-screening zu detektieren.

In einer bevorzugten Ausführungsform der Erfindung werden die Partikel ohne Abtrennung aus der Probe analysiert. Es sind somit keine einzelnen Schritte zur Abtrennung erforderlich, die eine zusätzliche Fehlerquelle sein können. Somit führt dieses bevorzugte Verfahren zu einer Verbesserung und Leistungssteigerung gegenüber den bekannten, wie auch zu einer erhöhten Zuverlässigkeit, der Beseitigung von Fehlern und daher insgesamt zu einer Qualitätshebung. Der Verzicht auf Separationsschritte bietet außerdem die Möglichkeit der Rationalisierung, Automatisierung oder Miniaturisierung.

In einer weiteren bevorzugten Ausführungsform der Erfindung liegt der K_{d}-Wert der Proteinwechselwirkung im Bereich zwischen 10⁻⁸ M und 10⁻³ M, bevorzugt 10⁻⁷ M und 10⁻⁴ M, besonders bevorzugt 10⁻⁶ M und 10⁻⁵ M, insbesondere bei ca. 10⁻⁶ M. Durch den K_{d}-Wert wird die Protein-Protein-Interaktion bzw. die Assoziierung zwischen den beiden Proteinen über das Massenwirkungsgesetz bestimmt. Der K_{d}-Wert ist reziprok zur Affinität, die die Stärke der Bindung zwischen den Proteinen kennzeichnet.

In einer weiteren besonders bevorzugten Ausführungsform der Erfindung ist das erste Protein Calcineurin und das zweite Protein NFAT. Bisher stand kein geeignetes Verfahren zu Verfügung, mit dem die Calcineurin-NFAT-Wechselwirkung in Form einer Protein-Protein-Bindung einfach, sicher und vor allem schnell bestimmt werden konnte. Vorteilhafterweise lässt sich mit dem erfindungsgemässen Verfahren die Protein-Protein-Wechselwirkung von Calcineurin mit NFAT bzw. mit einer NFAT-Docking-Domäne ganz spezifisch detektieren und weiterhin können Substanzen, die die Calcineurin-NFAT-Wechselwirkung modifizieren, mit diesem Verfahren einfach, sicher und effizient detektiert werden.

Calcineurin und NFAT haben eine große Bedeutung in T-Zellen. Bei der Antigen-spezifischen Stimulation von T-Zellen kommt es über den T-Zell-Rezeptor (TCR) zur Aktivierung verschiedener Proteine und Botenstoffe. Ein wichtiger aktivierender Botenstoff bei der TCR-Signalkette ist der zytosolische Calciumspiegel. Wird er erhöht, d. h. ist mehr Calcium im Zytosol, so wir die Phosphatase Calcineurin aktiviert. Diese wiederum dephosphoryliert, dann den Transkriptionsfaktor NFAT, der in den Kern wandert und dort die Transkription von wichtigen Zytokinen und anderen Proteinen, die für eine Immunantwort unerlässlich sind, bewirkt. Wird die Calcineurinaktivität blockiert, z. B. durch die immunsuppressiven Arzneimittel CyclosporinA (CsA) und Tacrolimus (FK506), so führt dies zur Verhinderung einer Immunanwort, was bei Transplantationspatienten erwünscht ist, da es eine Abstoßungsreaktion verhindert.

Vorteilhafterweise ist es mit dem erfindungsgemässen Verfahren möglich, Inhibitoren, die die Calcineurin-NFAT-Wechelwirkung blockieren, zu analysieren. CsA und FK506 hemmen die generelle Phosphataseaktivität von Calcineurin und somit auch die Dephosphorylierung von anderen Calcineurinsubstraten außer NFAT. Dies könnte eine der Ursachen für eine starke Toxizität bei Langzeitapplikation der beiden Drugs sein. Ein Arzneimittel, das nur die Aktivierung von NFAT verhindert und keinen Effekt auf die Dephosphorylierung anderer Calcineurinsubstrate hat, sollte spezifischer die Immunanwort hemmen and damit wahrscheinlich weniger Nebenwirkungen als CsA und FK605 haben. Eine geeignete Möglichkeit dies zu erzielen, kann die Blockierung der CaN-NFAT Bindung sein, denn ihre Protein-Protein-Wechselwirkung ist eine Vorbedingung für die Dephosphorylierung von NFAT durch CaN.

Eine bevorzugte Untersuchung der Calcineurin-NFAT-Wechselwirkung erfolgt beispielsweise so, dass fluoreszenzmarkiertes Calcineurin als FITC-Calcineurin mit einem biotinylierten 17mer optimierten Bindungspeptid von NFAT als biot-VIVIT oder anderen Bindungssequenzen inkubiert wird (siehe Beispiel). Das biot-VIVIT wird dann an Straptavidin-beschichteten Partikeln beispielsweise Streptavidin gecoatete Sepharosebeads gebunden. Das an VIVIT assoziierte Calcineurin wird in einem Durchflusszytometer über die partikelassoziierte Fluoreszenz nachgewiesen. Dem Fachmann sind geeignete Durchflusszytometer bekannt, beispielsweise "Facs Calibur" oder Luminexgeräte.

In Gegenwart von Verbindungen, die die Wechselwirkung bzw. die Calcineurin-VIVIT-Bindung inhibieren, werden keine oder wenige FITC-Calcineurin-Strukturen an den Partikeln nachgewiesen. Sofern Substanzen zugegeben werden, die die Wechselwirkung zwischen Calcineurin und VIVIT fördern, sind demgemäss höhere Fluoreszenzwerte an den Partikeln detektierbar.

Selbstverständlich ist es auch möglich, statt dem biotinylierten 17mer optimierten Bindungspeptid biot-VIVIT andere optimierte Bindungspeptide von NFAT bzw. Bindungspeptide von anderen Proteinen oder Peptiden zu verwenden. Beispielsweise können auch biot-AKAP79 bzw. biot-NFAT-B Peptide (siehe Beispiel) eingesetzt werden. Zu Kontrollzwecken kann auch Biot-TIVIV verwendet werden.

In einer weiteren bevorzugten Ausführungsform der Erfindung wird das erste Protein mit GFP, Dsred, Cy5 und/oder PE markiert. Als Fluoreszenzfarbstoffe können weiterhin eingesetzt werden: Dansylchlorid, Fluoresceinisothiocyanat, 7-Chlor-4-nitrobenzoxadiazol, Pyrenbutyrylessigsäure-anhydrid, N-Iodoacetyl-N'-(5-sulfonsäure-1-naphthyl)-ethylendiamin, 1-Anilino-naphthalen-8-sulfonat, 2-Toluidinonaphthalen-6-sulfonat, 7-(p-Methoxybenzyl-amino)4-nitro-benz-2-oxa-1,3-diazol, Formycin, 2-Aminopurinribonukleosid, Ethenoadenosin, Benzoadenosin, α- und β-Parinarsäure und/oder Δ^{9,11,13,15} Octaecatetraensäure, Cadmiumselenit-Kristalle einer oder unterschiedlicher Größen und andere; aber auch Übergangsmetallkomplexe, die folgende Substanzen enthalten: Ruthenium (II), Rhenium (I) oder Osmium und Iridium als Zentralatom und Diiminliganden; phosphoreszierende Prophyrine mit Platin, Palladium, Lutetium oder Zinn als Zentralatom; phosphoreszierende Komplexe der Seltenerden wie Europium, Dysprosium oder Terbium; phosphoreszierende Kristalle wie Rubin, Cr-YAG, Alexandrit oder phosphoreszierende Mischoxide wie Magnesiumfluorogermanat bzw. Cadmiumselenit-Kristalle, Fluorescein, Aminofluorescein, Aminomethylcoumarin, Rhodamin, Rhodamin 6G, Rhodamin B, Tetramethylrhodamin, Ethidiumbromid und/oder Acridinorange.

Als Fluoreszenzfarbstoffe können außerdem folgende Stoffe eingesetzt werden:
Ruthenium(II)-(tris-4,7-diphenyl-1,10-phenanthrolin)/HPTS
Ruthenium(II)-(tris-4,7-diphenyl-1,10-phenanthrolin)/Fluorescein
Ruthenium(II)-(tris-4,7-diphenyl-1,10-phenanthrolin)/Rhodamin B
Ruthenium(II)-(tris-4,7-diphenyl-1,10-phenanthrolin)/Rhodamin B-octadecylester
Ruthenium(II)-(tris-4,7-diphenyl-1,10-phenanthrolin)/Hexadecyl-Acridinorange
Europium(III)-tris-theonyl-trifluormethylacetonat/Hydroxymethylcoumarin
Platin(II)-tetraphenylporphyrin/Rhodamin B-octadecylester
Platin(II)-tetraphenylporphyrin/Rhodamin B
Platin(II)-tetraphenylporphyrin/Naphtofluorescein
Platin(II)-tetraphenylporphyrin/Sulforhodamin 101
Platin(II)-octaethylporphyrin/Eosin
Platin(II)-octaethylporphyrin/Thionin
Platin(II)-octaethylketoporphyrin/Nilblau
Cr(III)-YAG/Nilblau und
Cr(III)-YAG/Naphtofluorescein.
Aminocoumarin/Aminofluorescein
Aminocoumarin/Rhodamin 6G
Aminocoumarin/Tetramethylrhodamin
Aminocoumarin/Acridinorange
Aminofluorescein/Rhodamin 6G
Aminofluorescein/Tetramethylrhodamin und/oder
Aminofluorescein/Ethidiumbromid.

Das zweite Protein wird bevorzugt mit Tags kombiniert ausgewählt aus der Gruppe umfassend: His-Tag (z. B. 4-7 Histidinreste), Flag-Tag (z. B. Octapeptid), Strep-Tag, T7-Tag (z. B. 11 N-terminale Aminosäuren des T7Gen10-Proteins), S-Tag, CBP (Calmodulin-Binde-Peptid), MBP (Maltose-Binde-Peptid), Neb, Protein A, GST, PinPoint-Tag, Thioredoxin, PET, PMal, Biotin-Tag und andere. Selbstverständlich ist es möglich, die Tags als anti-Tags einzusetzen und umgekehrt, d. h. bei einem Biotin-Tag ist das anti-Tag Streptavidin, bei einem Streptavidin-Tag ist das anti-Tag Biotin.

In einer weiteren bevorzugten Ausführungsform der Erfindung werden als das erste und/oder zweite Protein die Peptide dieser eingesetzt, die mindestens eine Bindungs-Domäne der Proteine oder ein Fragment hiervon umfassen. Das heißt, es kann in einer bestimmten Ausführungsform der Erfindung vorteilhaft sein, anstelle des ersten oder des zweiten kompletten Proteins ein Peptid zu verwenden, welches aus dem Protein generiert wurde und mindestens eine Bindungs-Domäne oder ein Fragment hiervon umfasst. Dem Fachmann ist bekannt, das für die Wechselwirkung zweier Proteine die Primär-, Sekundär-, Tertiär- und/oder Quartierstruktur verantwortlich sind. Auf allen diesen Ebenen ist es möglich, die Wechselwirkung zweier Proteine bzw. Peptide zu beeinflussen. Auch wenn bei der Wechselwirkung von zwei Proteinen die Tertiär- bzw. Quartierstruktur von Bedeutung sind, so können insbesondere spezifische Teilabschnitte bzw. Peptidmotive der Proteinen - die Bindungs-Domänen oder Docking-Domänen - die Wechselwirkung sehr spezifisch beeinflussen. Es ist beispielsweise möglich, dass durch Mutationen in diesen Peptidmotiven die Domänen so nachhaltig modifiziert werden, dass keine Interaktion mehr zwischen den Proteinen bzw. Peptiden möglich ist. Für eine genaue Analyse der Wechselwirkung zwischen zwei Proteinen kann es daher vorteilhaft sein, die entsprechenden modifizierten oder teilweise modifizierten Bindungs-Domänen einzusetzen.

Weiterhin kann es bevorzugt sein, wenn diese Bindungs-Domänen, oder auch andere Bereiche des Proteins, durch verschiedene chemische, biologische oder physikalische Modifikationen in ihrem Bindungsverhalten verändert werden, vorteilhafterweise so, dass ihr Bindungsverhalten optimiert wird. Bekannte Verfahren hierzu sind beispielsweise die Aminosäuredeletion, -substitution, -addition und/oder - insertion oder der Einbau von nicht-Aminosäurebausteinen. Derartige Verfahren können selbstverständlich mit Modifikationen kombiniert werden. Jede Veränderung der Aminosäurestruktur kann im Sinne der Erfindung eine Optimierung der Bindungs-Domäne sein. Dies schließt u. a. den Einbau von artifiziellen bzw. modifizierten Aminosäuren oder nicht-Aminosäurebausteine in die Bindungs-Domäne oder in andere Bereiche des Proteins ein.

In einer weiteren bevorzugten Ausführungsform handelt es sich bei den anti-Tag, insbesondere Streptavidin-beschichteten Partikel um Sepharose-, Polystyren-, Agarose- und/oder Latexpartikel. Dem Fachmann sind weitere Partikel bekannt, die er als sogenannte Beads in der Durchflusszytometrie einsetzen kann.

Die Erfindung betrifft auch einen Kit, der ein erstes markiertes Protein, ein zweites Tag-kombiniertes Protein und/oder anti-Tag-beschichtete Partikel, ggf. zusammen mit einer Information zum Kombinieren der Inhalte des Kits umfasst. Dieser Kit kann bevorzugt angewendet werden, um die Wechselwirkung zwischen dem ersten und dem zweiten Protein zu detektieren. Hierzu kann es vorteilhaft sein, dass das erste und/oder zweite Protein ein in der Bindungssequenz/-domäne oder anderweitig optimiertes Protein und/oder Peptid darstellt.

Im Folgenden soll die Erfindung an Hand eines Beispiels erläutert werden, ohne auf dieses Beispiel beschränkt zu sein.

### Beispiel

### Material/Methoden

### Material

Rekombinantes menschliches CaN wurde durch Überexpression in E. coli und anschließende Reinigung gewonnen. Die verwendeten Peptide
VIVIT (GMAGPHPVIVITGPHEE),
biot-VIVIT (biotinyl-GMAGPHPVIVITGPHEE),
biot-TIVIV (biotinyl-EEHPGTIVIVPHPGAMG),
AKAP79 (GGRRKRSESSKQQKPLE),
biot-AKAP79 (biotinyl-GGRRKRSESSKQQKPLE) sowie
biot-NFAT-B Peptid (4 *µ*M) (Sequenz: KGGFCDQYLAVPQHPYQWAK)
wurden durch Festphasensynthese hergestellt.

Die Streptavidin gecoateten Sepharosebeads waren von Amersham Biosciences. Alle anderen verwendeten Chemikalien wurden von Sigma bezogen.

Der Assaypuffer war wie folgt zusammengesetzt: 25mM Tris/HCl, 3mM MgCl₂, 1mM EGTA, 0,5mM DTT oder TCEP, pH 7,4. Wenn nicht anders angegeben, wurde der Assaypuffer noch mit 0,05% BSA (PAA laboratories) versetzt.

### FITC-Markierung des rhCalcineurin

Rekombinantes menschliches CaN wurde in Boratpuffer aufgenommen und mit FITC (Sigma) in einem molaren Verhältnis von 1:12 für 1h auf Eis und vor Licht geschützt inkubiert. Das ungebundenen FITC wurde dann über eine PD10 Säule (Amersham-Pharmacia) abgetrennt.

### Bindungsassay

Zu rekombinantem menschlichen FITC-CaN (25 nM), das in Assaypuffer mit 0,05% BSA mit biotinyliertem VIVIT-Peptid (300 nM) bzw. NFAT-B Peptid (4 *µ*M) für 10 min lichtgeschützt auf Eis inkubierte, wurden 50000 beads in 10 *µ*l Asaypuffer pipettiert. Nach weiteren 10 min Inkubation wurde das Reaktionsgemisch (110 *µ*l) im Durchflusszytometer "FacsCalibur" (BD Biosciences) gemessen. Bei der Testung von Substanzen auf ihre Fähigkeit, die CaN-VIVIT Bindung zu blockieren, wurde ein zusätzlicher Inkubationsschritt dem o.g. Assay vorgeschaltet. Dafür wurde 1 *µ*l Inhibitor (in DMSO) zu FITC-CaN in Assaypuffer (94 *µ*l) pipettiert und anschließend für 30 min lichtgeschützt auf Eis belassen. Die Auswertung der Meßdaten erfolgte mit der Software CellQuest (BD Biosciences).

### Ergebnisse

### CaN wird über das VIVIT-peptid an Beads gebunden

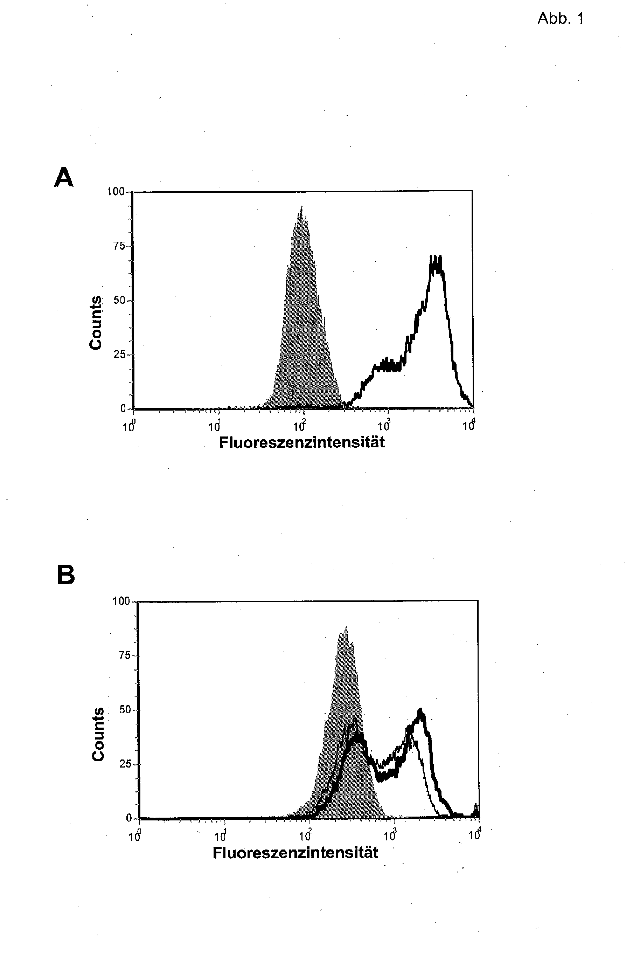

Bei Inkubation von FITC markiertem CaN mit bzw. ohne biotinyliertem VIVIT-Peptid konnte nach Zugabe von Streptavidin-Beads im Durchflusszytometer eine deutlich unterschiedliche mittlere Fluoreszenzintensität nachgewiesen werden (Abb. 1) . Da bei der Durchflusszytometry nur die partikelassoziierte Fluoreszenz erfasst wird, war kein Zentrifugations- oder Waschschritt notwendig. Die erhöhte Fluoreszenzintensität in Gegenwart von VIVIT-Peptid zeigte, dass FITC-CaN an das VIVIT-Peptid bindet. Um die Spezifität der CaN-VIVIT-Bindung zu prüfen, wurden verschiedene Tests zur Untersuchung unspezifischer Bindungen durchgeführt.

### Spezifität der Bindung

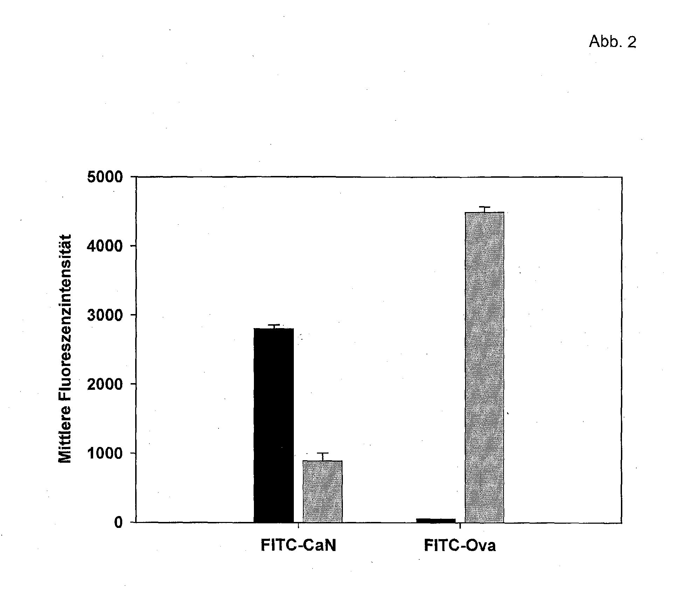

Das als Kontrollprotein verwendete FITC markierte Ovalbumin, das mit dem VIVIT-Peptid nicht interagiert, zeigte auch in dem verwendeten Bindungsassay mit VIVIT-Peptid keine erhöhte Fluoreszenzintensität (Abb. 2). Im Gegensatz dazu bindet ein Peptid des Ankerproteins AKAP79, das für seine unspezifische Bindung bekannt ist, sowohl FITC-CaN als auch FITC-Ovalbumin.

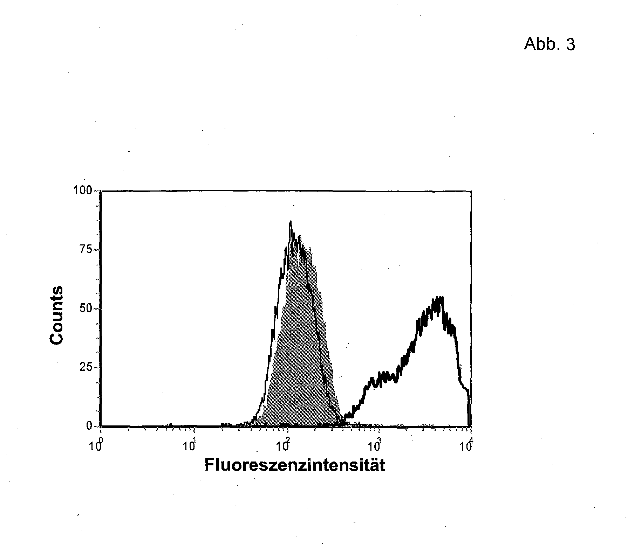

Um nachzuweisen, daß nicht unspezifische Bindungen in den Peptidseitenketten die Bindung von CaN im Assay bewirken, wurde das biotinylierte Umkehrpeptid (TIVIV-Peptid) anstelle von biot-VIVIT-Peptid eingesetzt. Abbildung 3 zeigt die Spezifität der CaN-VIVIT Bindung, da nur mit biotinyliertem VIVIT-Peptid und nicht mit biot-TIVIV eine erhöhte Fluoreszenzintensität gemessen wurde.

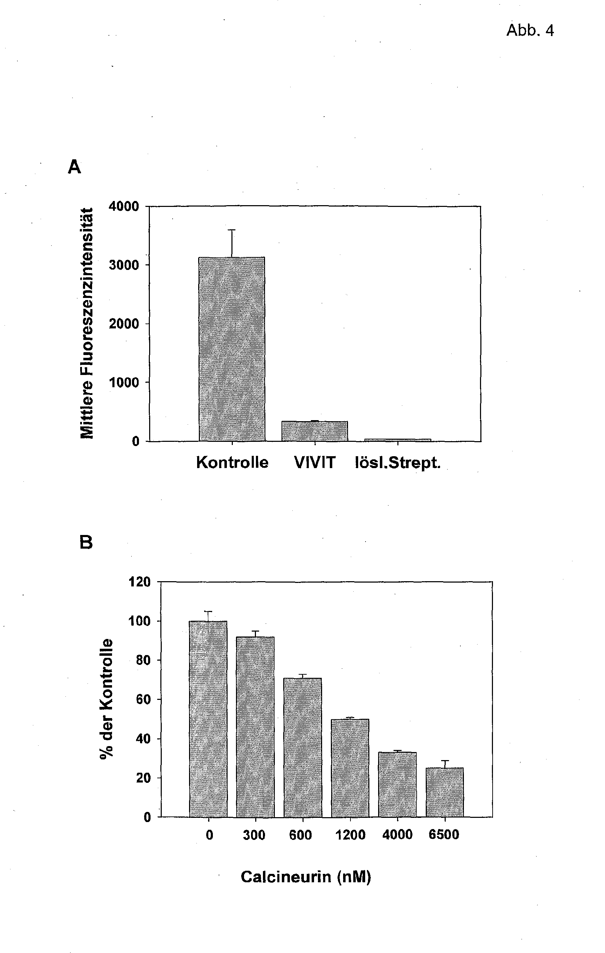

Durch spezifische Konkurrenz (Kompetierung) von zugegebenen Verbindungen mit Komponenten eines Assays lassen sich ebenfalls Aussagen über die Spezifität von Bindungen treffen. In Abbildung 4 ist dargestellt, daß alle getesteten Assaypartner kompetierbar waren, da in Gegenwart eines Überschusses der Konkurrenzpartner kein beadassoziiertes FITC-CaN mehr nachweisbar war: lösliches Streptavidin (330 nM) verdrängt beadgebundenes Streptavidin, nichtbiotinyliertes VIVIT (1500 nM) verdrängt biotinyliertes VIVIT (Abb. 4A) und nicht markiertes CaN verdrängt FITC-CaN konzentrationsabhängig (Abb. 4B).

### Optimierung der Versuchsbedingungen

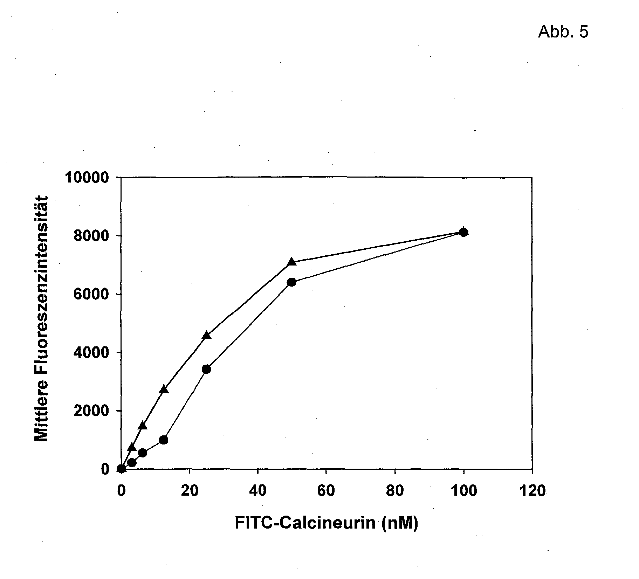

Die in Abbildung 5 dargestellte Abhängigkeit der mittleren Fluoreszenzintensität von der verwendeten CaN Konzentration zeigte in Gegenwart von biot-VIVIT (300 nM) und 0,1% BSA im Assaypuffer eine weitgehende Linearität im CaN-Konzentrationsbereich 0-50 nM. Dies weist nach, daß unter diesen Bedingungen die Fluoreszenzintensität im Bindungsassay mit der Menge an beadgebundenem FITC-CaN korreliert. Die Verwendung von 0,1% oder 0,05% BSA im Assaypuffer führte nicht nur zu einer besseren linearen Abhängigkeit der Fluoreszenzintensität von der eingesetzten CaN Konzentration (Abb. 5), sondern auch zu einer deutlich verminderten unspezifischen Bindung von FITC-CaN an die Beads.

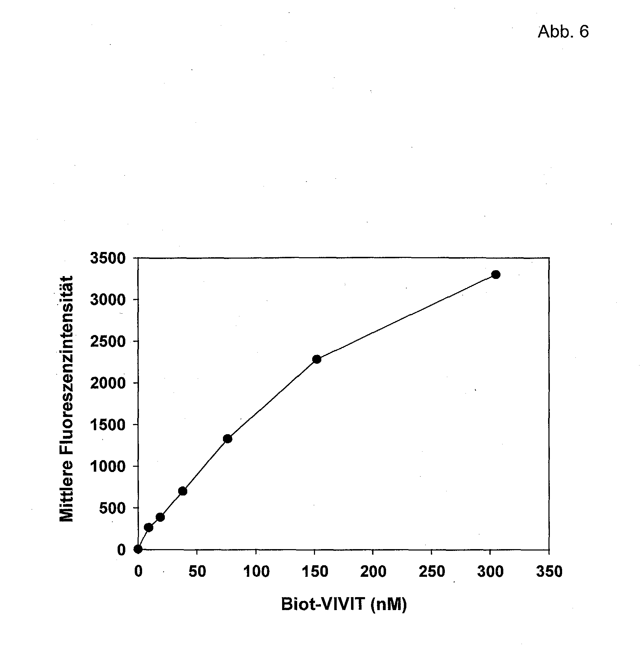

Bei konstanter FITC-CaN Konzentration (25 nM) korreliert die mittlere Fluoreszenzintensität auch mit der eingesetzten biot-VIVIT Konzentration und zeigt im Bereich von 0-100 nM eine lineare Abhängigkeit (Abb. 6). Im Assay wurde eine Konzentration von 300 nM biot-VIVIT eingesetzt, um einen leichten Überschuß an Peptid zu haben.

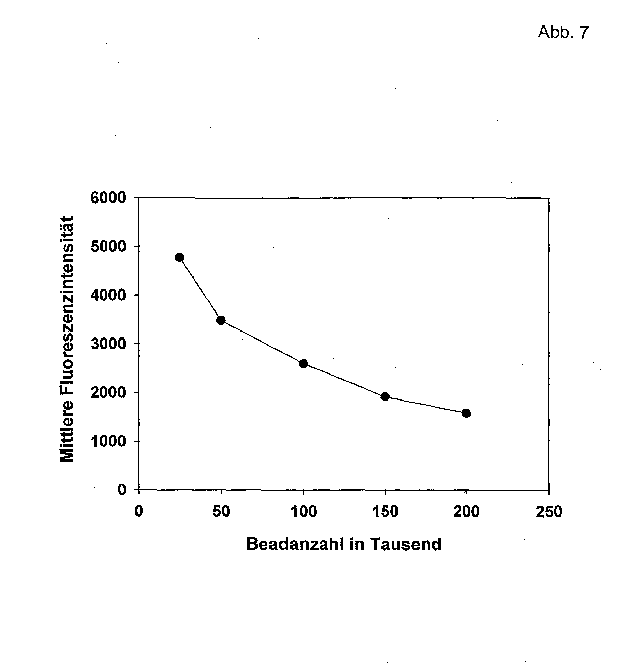

Bei konstanter FITC-CaN (25 nM) und biot-VIVIT Konzentration (300 nM) führte eine Erhöhung der eingesetzten Beadanzahl im Reaktionsvolumen von 110 *µ*l erwartungsgemäss zu einer verminderten Fluoreszenzintensität pro bead. Als optimale Beadmenge pro Ansatz wurden 50000 Beads ermittelt. Zum einen wurde damit eine gute mittlere Fluoreszenzintensität erzielt und zum anderen standen genügend Beads zur Messung von 10000 Ereignissen im Durchflusszytometer zur Verfügung (Abb. 7).

Von verschiedenen getesteten Puffersystemen erwies sich der verwendete Assaypuffer (25mM Tris/HCl, 3mM MgCl₂, 1mM EGTA, 0,5mM DTT oder TCEP, pH 7,4) mit 0,05% oder 0,1% BSA am geeignetsten.

Bei Testung der notwendigen Inkubationszeit von FITC-CaN mit biotinyliertem VIVIT-Peptid erwiesen sich 10 min als ausreichend, da längere Zeiten keine Erhöhung der Bindung bewirkten.

### Screening von Substanzen

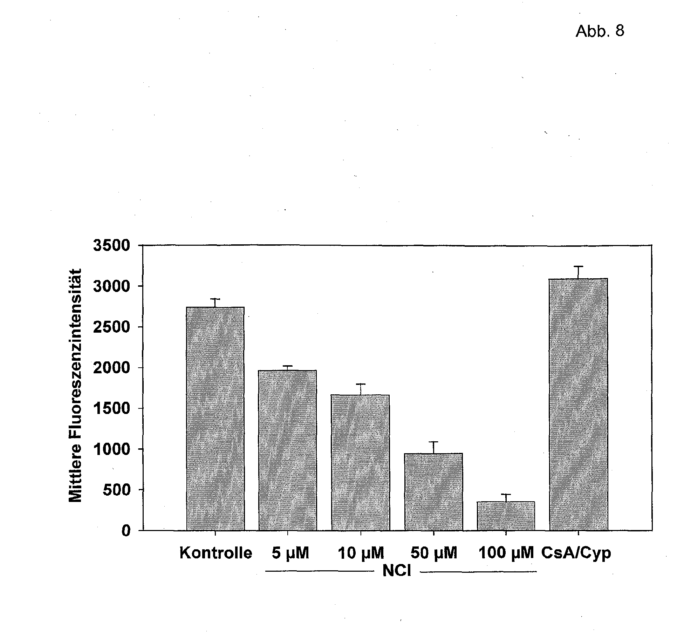

Zur Testung von verschiedenen niedermolekularen immunsuppressiven Substanzen auf ihre Fähigkeit, die CaN-VIVIT Bindung zu blockieren, wurde ein zusätzlicher Inkubationsschritt von FITC-CaN mit der entsprechenden Verbindung vorgeschaltet. Als negative Kontrolle wurde der immunsuppressive Komplex CsA/Cyclophilin18 (Cyp) getestet, der bekanntermaßen die Calcineurinaktivität nicht aber die CaN-NFAT Bindung hemmt. In dem Bindungsassay wird dieser Befund bestätigt, da CsA/Cyp die FITC-CaN Bindung an die beads über das biot-VIVIT nicht hemmt (Abb. 8).

Das Screening von verschiedenen immunsuppressiven Substanzen zeigte bisher bei 4 Verbindungen eine verminderte mittlere Fluoreszenzintensität, die auf eine Blockierung der CaN-VIVIT Bindung hindeutet. Abbildung 9 stellt die konzentrationsabhängige Verdrängung von FITC-CaN von VIVIT durch die Substanz NCI dar. Dieser im Bindungsassay gefundene Effekt von NCI konnte im Pull-down Assay bestätigt werden.

Es konnte nachgewiesen werden, daß der Assay auch in Gegenwart der CaN Aktivatoren Ca²⁺ und CaM zuverlässige Ergebnisse liefert. Dies ist von Bedeutung, da einige Inhibitoren möglicherweise nur an der Konformation der aktiven Form von CaN binden können.

### Legenden

### Abb. 1:

### (A) Bindung von FITC-CaN über VIVIT an Beads

FITC markiertes CaN (25 nM) bindet an biot-VIVIT-Peptid (300 nM) und zeigte nach Zugabe von Streptavidin-Beads (50000) im Durchflusszytometer eine höhere mittlere Fluoreszenzintensität (schwarze Linie) als die Kontrolle ohne biot-VIVIT-Peptid (gefülltes Histogramm).

### (B) Bindung von FITC-CaN über NFAT-B-Peptid an Beads

FITC markiertes CaN (25 nM) bindet an biot-NFAT-B-Peptid (4*µ*M) und zeigte sowohl ohne Ca²⁺/Calmodulin (dünne schwarze Linie), als auch mit Ca²⁺/Calmodulin (dickere schwarze Linie) eine höhere mittlere Fluoreszenzintensität als die Kontrolle ohne biot-NFAT-B-Peptid (gefülltes Histogramm).

### Abb. 2:

### Biot-VIVIT bindet spezifisch FITC-CaN im Assay

Das verwendete biot-VIVIT-Peptid (300 nM) (schwarze Balken) bindet spezifisch FITC-CaN (25 nM) aber nicht das Kontrollprotein FITC-Ovalbumin (25 nM). Dagegen bindet ein Peptid des Ankerproteins AKAP79 (graue Balken), das für seine unspezifische Bindung bekannt ist, sowohl FITC-CaN als auch FITC-Ovalbumin.

### Abb. 3:

### Biot-TIVIV (Umkehrpeptid) bindet kein FITC-CaN an Beads

Das biotinylierte Umkehrpeptid (biot-TIVIV-Peptid) (dünne schwarze Linie) zeigte im Assay keine erhöhte Fluoreszenzintensität gegenüber der Kontrolle ohne jegliches Peptid (gefülltes Histogramm). Als Positivkontrolle diente das biot-VIVIT-Peptid (schwarze dickere Linie), das eine erhöhte Fluoreszenzintensität gegenüber der Kontrolle aufweist.

### Abb. 4:

### Spezifische Kompetierung von Assaykomponenten

(A) Eine beadassoziierte spezifische Bindung von FITC-CaN über biot-VIVIT war nicht mehr nachweisbar, wenn ein 50 facher Überschuß von nichtbiotinyliertem VIVIT das FITC-CaN bindet oder lösliches Streptavidin (330 nM) an den biot-VIVIT/ FITC-CaN Komplex bindet und damit das beadgebundenes Streptavidin verdrängt. (B) Nicht FITC markiertes CaN verdrängt FITC-CaN konzentrationsabhängig von der VIVIT Bindungsstelle.

### Abb. 5:

### Die Fluoreszenzintensität korreliert mit der CaN Konzentration

Die mittleren Fluoreszenzintensität korreliert mit der verwendeten CaN Konzentration in Gegenwart von biot-VIVIT (300 nM) und 0,1% BSA im Assaypuffer (Dreiecke). Ohne BSA (Kreise) wird im unteren CaN Konzentrationsbereich keine Linearität erreicht.

### Abb. 6:

### Die Fluoreszenzintensität korreliert mit der Biot-VIVIT Konzentration

Die mittlere Fluoreszenzintensität korreliert mit der verwendeten biot-VIVIT Konzentration in Gegenwart von FITC-CaN (300 nM) und 0,1% BSA im Assaypuffer.

### Abb. 7:

### Die Fluoreszenzintensität korreliert negativ mit der Beadanzahl

Eine Erhöhung der eingesetzten Beadanzahl bei konstanter FITC-CaN (25 nM) und biot-VIVIT Konzentration (300 nM) und im gleichen Reaktionsvolumen führte zu einer verminderten Fluoreszenzintensität pro bead.

### Abb. 8:

### Identifikation eines Inhibitors der CaN-NFATepitop Bindung

Eine konzentrationsabhängig verminderte Fluoreszenzintensität in Gegenwart der immunsuppressiven Substanz NCI weist auf eine verminderte Interaktion von FITC-CaN und biot-VIVIT hin. Die Negativkontrolle, der CsA/Cyp Komplex, verändert die Fluoreszenzintensität nicht.

## Patentansprüche

1. Verfahren zur Detektion einer transienten
Protein-Protein-Wechselwirkung in einer Probe
**dadurch gekennzeichnet, dass**
ein erstes Protein markiert, ein zweites Protein mit einem Tag kombiniert, die Proteine mit anti-Tag beschichteten Partikel in Kontakt gebracht, inkubiert und durchflusszytometrisch analysiert werden und die Detektion der Markierung auf den Partikeln ein Hinweis auf die Protein-Protein-Wechselwirkung ist.

2. Verfahren nach Anspruch 1
**dadurch gekennzeichnet, dass**
die Partikel ohne Abtrennung aus der Probe analysiert werden.

3. Verfahren nach Anspruch 1 oder 2,
**dadurch gekennzeichnet, dass**
das erste Protein mit FITC, GFP, Dsrde, Cy5 und/oder PE markiert und/oder das zweite Protein mit His-Tag, Flag-Tag, Strep-Tag, T7-Tag, S-Tag, CBP, MBP, Neb, Protein A, GST-Tag, PinPoint-Tag, Thioredoxin, PET, PMal und/oder Biotin-Tag kombiniert werden.

4. Verfahren nach einem der vorhergehenden Ansprüche
**dadurch gekennzeichnet, dass**
das anti-Tag ausgewählt ist aus der Gruppe umfassend Streptavidin, Glutathion, Biotin, Nickel-NTA, Cellulose, Amylose, Thiobond, Avidin, und/oder Immunglobulin.

5. Verfahren nach einem der vorhergehenden Ansprüche
**dadurch gekennzeichnet, dass**
als Partikel Sepharose-, Polystyren-, Agarose- und/oder Latexpartikel eingesetzt werden.

6. Verfahren nach einem der vorhergehenden Ansprüche
**dadurch gekennzeichnet, dass**
der K_{d}-Wert der Protein-Protein-Wechselwirkung im Bereich zwischen 10⁻⁸ M und 10⁻³ M, bevorzugt zwischen 10⁻⁷ M und 10⁻⁴ M, besonders bevorzugt zwischen 10⁻⁶ M und 10⁻⁵ M und ganz besonders bevorzugt bei im wesentlichen 10⁻⁶ M.

7. Verfahren nach einem der vorhergehenden Ansprüche
**dadurch gekennzeichnet**, das
als erstes Protein Calcineurin und/oder als zweites Protein NFAT verwendet werden.

8. Verfahren nach einem der vorhergehenden Ansprüche
**dadurch gekennzeichnet, dass**
als das erste und/oder zweite Protein Peptide dieser eingesetzt werden, die eine Bindungsdomäne der Proteine umfassen.

9. Verfahren nach einem der vorhergehenden Ansprüche
**dadurch gekennzeichnet, dass**
die Bindungsdomäne durch Aminosäuredeletion, -substitution, -addition, -insertion und/oder den Einbau von nicht-Aminosäurebausteinen optimiert werden.

10. Kit umfassend ein erstes markiertes Protein, ein zweites Tag-kombiniertes Protein und/oder anti-Tag-beschichtete Partikel gegebenenfalls zusammen mit einer Information zum Kombinieren der Inhalte des Kits.

11. Verwendung des Kits nach dem vorhergehenden Anspruch zur Detektion einer Wechselwirkung zwischen dem ersten und dem zweiten Protein.

12. Verwendung des Kits nach Anspruch 10 zur Durchführung des Verfahrens nach einem der Ansprüche 1-9.
